# EUROPEAN PATENT SPECIFICATION

(11) **EP 2 099 377 B1**
(45) Date of publication and mention of the grant of the patent: **30.10.2013**
(21) Application number: 07726045.3
(22) Date of filing: 18.06.2007
(51) Int. Cl.: A61B 19/00

(54) **A method and system for integrating image information from an external source**
Verfahren und System zur Integration von Bildinformation einer externen Quelle
Procédé et système d'intégration d'information d'un image provenant d'une source extérieure

(43) Date of publication of application: 16.09.2009
(73) Proprietor: BrainLAB AG, 85622 Feldkirchen (DE)
(72) Inventor: WOERLEIN, Swen, 81479 Munich (DE); TIEHMANN, Ingmar, 80686 Munich (DE); MATHIS, Martin, 85653 Aying-Grosshelfendorf (DE); WEISSENBORN, Anke, 90419 Nürnberg (DE)
(74) Representative: Schwabe - Sandmair - Marx
(86) International application number: PCT/EP2007/005334
(87) International publication number: WO 2008/154935

(56) References cited:
- EP-A- 1 695 670
- WO-A-01/93770
- DE-U1-202005 021 111
- US-A- 4 722 056
- US-A- 5 715 836
- US-B1- 6 314 311

## Description

The present invention relates to a surgical microscope with integrated structured illumination and to reference display systems that may be used to project or display additional information, as e.g. provided from three-dimensional imaging or beamer devices, on the surface of an object viewed by an operating microscope e.g. during an operative procedure on a patient, e.g. by directly projecting optical information obtained e.g. from an MR or CT scan onto the patient or an object to be viewed using the microscope.

Several technologies, as e.g. computer tomographic imaging, stereotaxy, and microsurgery have rapidly evolved as important tools in clinical neurosurgery. Advances in imaging techniques (CT, PET, MRI ...) now provide three-dimensional information about anatomic and pathologic structures previously not realized. Stereotaxy, through use of a three-dimensional coordinate system and a mechanical frame, now allows the delivery of a probe to an intracranial target with great accuracy. The operating microscope provides the magnification and illumination to enable surgeons to work with significantly greater precision and safety.

At present, a neurosurgeon's ability to perform intracranial procedures for tumor, vascular disease or functional disorder is dependent upon his mental integration of the visualized operative field with his knowledge of neuroanatomy and the available radiologic studies such as CT or MR scans.

Conventional CT scans are oriented transversely to the body axis and as the operative approach is rarely along the axis of conventional scanning, the ability to reconstruct a CT scan to match the surgical perspective is highly appealing.

CT scanning and reconstruction have become an integral and important part of modem medicine; the procedure used involves primarily image processing software and computer graphics. Adaptation of stereotactic technique to CT technology has been approached in a number of ways. One technique utilizes an adapted metal frame fixed to the patient's head at the time of scanning. Stereotactic coordinates, relating the target position of CT-demonstrated pathology to the stereotactic instrument, are generated directly from the scans and the patient is then transported to the operating room. Other developed techniques are adequate but often more cumbersome. All of these enable stereotactic procedures generally characterized by "blind" insertion of needle-like instruments through small openings utilizing previously obtained CT-determined landmarks. Earlier developments have not generally been amenable to "open" procedures such as craniotomy for tumor or vascular disease and, as previously noted, do not allow access to CT data after selection of a target. The CT information utilized is limited to the coordinates of a point. All instruments select a target and set the instrument accordingly; the present invention, operating in reverse, allows free positioning of the microscope with subsequent stereotactic positioning the additional data, as e.g. CT data or MR data, directly on an object or patient.

The operating microscope has been incorporated into CT stereotactic work described in two formal articles: "A Stereotactic Approach to Deep-Seated Central Nervous System Neoplasms Using the Carbon Dioxide Laser" (Surg-Neurol, 15: 331-334, 1981, Kelly et al.); and "A Microstereotactic Approach to Deep-Seated Arteriovenous Malformations," (Surg-Neurol, 17: 260-262, 1982, Kelly et al.). The Kelly et al. development has also employed surgical laser instrumentation and shows the feasibility of achieving a synthesis of technologies and the prospects of refining neurosurgical operative techniques. Their technique of linking the operating microscope and the stereotactic reference system requires utilization of a large stereotactic frame and does not employ a projection system.

Although the present system is discussed herein mostly in the context of neurosurgery, e.g. craniotomy, it is useful in other operating contexts.

US 6,466,815 B1 discloses a navigation apparatus comprising a navigation-related information generating section and a display section. The navigation-related information generating section measures the position and orientation of an object and a target in a three-dimensional space and generates navigation-related information to be used for navigating the object toward the target. The display section displays the navigation-related information generated by the navigation-related information generating section in any of different modes depending on the relationship of the position and orientation of the object and that of the target. A surgical operation image acquisition/display apparatus comprises an observation section, an image display section and a specifying section. The observation section includes a plurality of observation sections whose position and orientation is modifiable. The image display section is adapted to alternatively display any of the images obtained by the observation sections or synthetically combine and display the combined images. The specifying section specifies the image to be displayed to the image display section according to the position and orientation of the observation section.

US 6,577,080 B2 discloses methods and systems for signal processing for illumination control signals, which may include a decoder for decoding a combined signal and a connection for delivering a portion of the combined signal to an illumination source, which source is capable of generating an illumination condition from the portion of the combined signal. The system may also include an encoder for encoding the combined signal from an illumination control signal and a second signal. The illumination source may be an LED system that is controlled by a microprocessor to vary at least one of the color and intensity of the illumination produced by the illumination source in response to the illumination control signal.

US 5,769,861 discloses a method of localizing an instrument relative to three-dimensional corporeal data, particularly for stereotaxis, comprising the following steps: connecting internal markers fixedly to the body to define an intracorporeal, spatial reference system, implementing an analytical scan of the body including the internal markers to determine the positions of the three-dimensional corporeal data obtained from analytical scan in the intracorporeal reference system defined by the internal markers, implementing a referencing step, whereby position and orientation of the intracorporeal reference system defined by the internal markers relative to an extracorporeal reference system defined by external markers is determined and said external markers is located outside of the body in a fixed spatial relationship, determining the position and orientation of an instrument in the extracorporeal reference system with the aid of the external markers and a instrument markers attached to the instrument, and computing from said position and orientation in the extracorporeal reference system the position and orientation of the instrument in the intracorporeal reference system via the relationship between the intracorporeal and the extracorporeal reference systems known from the referencing step, as well as a markers device and a device for referencing.

US 6,351,659 B1 and US 6,859,660 B2 disclose a Neuro-navigation system comprising a reflector referencing system including passive reflectors and a marker system with markers or landmarks wherein the reflectors as well as the markers as regards their shape, size and material selection as well as their arrangement or attachment on the parts of the body to be operatively treated and on the surgical instruments are configured so that mapping their locations is substantially facilitated or is able to take place more accurately positioned by a computer/camera unit having a graphic display terminal as well as the operative treatment with the aid of this unit. Optionally a surgical microscope, an ultrasonic diagnostic system as well as a calibration procedure may be integrated in the Neuro-navigation system in accordance with the invention.

US 6,236,875 B1 discloses a system for use during a medical or surgical procedure on a body. The system generates an image representing the position one or more body elements during the procedure using scans generated by a scanner prior or during the procedure. The image data set has reference points for each of the body elements, the reference points of a particular body element having a fixed spatial relation to the particular body element. The system includes an apparatus for identifying, during the procedure, the relative position of each of the reference points of each of the body elements to be displayed. The system also includes a processor for modifying the image data set according to the identified relative position of each of the reference points during the procedure, as identified by the identifying apparatus, said processor generating a displaced image data set representing the position of the body elements during the procedure. The system also includes a display utilizing the displaced image data set generated by the processor, illustrating the relative position of the body elements during the procedure. Methods relating to the system are also disclosed. Also disclosed are devices for use with a surgical navigation system having a sensor array which is in communication with the device to identify its position. The device may be a reference frame for attachment of a body part of the patient, such as a cranial reference arc frame for attachment to the head on a spine reference arc frame for attachment to the spine. The device may also be a localization frame for positioning an instrument relative to a body part, such as a localization biopsy guide frame for positioning a biopsy needle, a localization drill guide assembly for positioning a drill bit, a localization drill yoke assembly for positioning a drill, or a ventriculostomy probe for positioning a catheter.

US 4,722,056 discloses a reference display system that receives information from an imaging system (e.g., a CT scanner or the like), that extracts or derives three-dimensional anatomical and/or pathological information about a part of a body (e.g., the brain or other organ) of a patient. The information is digitized in the imaging system and is introduced to a computer that is programmed to reformat the digitized information to provide as output electric signal representative of the digitized information. An optical display system (e.g., a cathode ray tube, CRT, and related circuitry) is connected to receive the output of the computer and is operable to present the reformatted information at a determined plane during an operative procedure. An operating microscope is freely located in the operative location relative to the patient during the operative procedure, the focal plane of the microscope establishing the determined plane. A way is provided to establish the spatial relationship among the imaging system, the patient, and the focal plane of the microscope; and a mechanism is provided to project the reformatted imaging system information into the optics and onto the focal plane of the operating microscope during the operative procedure, the reformatted image being displayed as an overlay upon the optical image of the body part on which the operation is being performed.

US 5,715,836 discloses a process for planning and monitoring a surgical operation. A desired image of the operation site is generated and an image of the planned section surface is generated, which image is superposed during the operation on an actual image of the operation site or is projected onto the operation site itself.

EP 1 695 670 A1 discloses a miniature laser projector which can be held by a user.

WO 01/93770 A1 relates to the guidance to a surgeon for guiding the tip of a surgical instrument along a previously defined optimum path subdivided into a plurality of sections. An image is performed on an exposed surface of the body upon which the surgeon is operating. The image is focused below the exposed surface and at the next intermediate point to be traversed along the path. The image provides the surgeon with vector information as to where he should direct the tip of the instrument. The image consists of three or more overlapping dots of lights from lasers which are directed at the intermediate point by motorized mountings under the control of a computer.

DE 20 2005 021 111 U1 discloses a microscope having a unit for illuminating a surface from a first direction, a unit for projecting an image onto a surface from a second direction and a microscope for viewing the surface from a third direction.

US 4,722,056 discloses a reference display system, wherein information from an imaging system is reformatted and the reformatted information is projected into the optics of an operating microscope.

The present invention integrates external or additional information, obtained e.g. from CT or MR, and computer technology and preferably stereotactic principles and the operating microscope, to develop a computer-based optical system for use during microneurosurgical procedures. This technique has the capability to incorporate the available computer technology with the operating room environment and many neurosurgical procedures.

An objective of the present invention is to provide a system that will project and display reconstructed CT images on at least part of the surface of an object preferably in the field of view of the operating microscope. The neurosurgeon will then see, for example, the outline of a tumor (reconstructed by a computer) superposed on the operative field.

This provides advantages, since e.g. no dependence on the surgeon's mental reorientation of CT scanner information exists. The information can be displayed such that it will not interfere with the neurosurgeon's procedure or require the reading of x-rays off a light screen some distance away. A computer-based anatomical atlas can be developed that will superpose on the operative field important, but otherwise unseen, structures such as normal neuronal pathways and nuclei and major vascular structures. The neurosurgeon can use the projected image(s) as a map accurately guiding operative procedures with greater precision than presently possible.

Another objective of the present invention is to provide a system that will superpose appropriately reformatted, three-dimensional imaging information on the object or patient by projecting the generated optical information on the object which is visualized through the operating microscope.

A still further objective is to provide the capability to present information of structure lying directly below the focal plane of the microscope by providing an optical information directly on the object, so the surgeon can find the location on the surface directly above the region of interest.

Another objective is to present to the surgeon accurate information on the boundary between different tissues e.g. by displaying on the object the boundary and/or by displaying different regions in different colours so the surgeon can locate such boundaries accurately; for example, the surgeon may be debulking a tumor and wish to know where the edge of the tumor is relative to normal brain tissue.

An additional objective is to overlay or project on the object being in the visual field of the microscope, generic information from an atlas of information about functional purposes of different regions of the brain.

The foregoing objectives are attained generally in a reference display system and method of integrating image information from an external source according to claim 1, 5.

The invention allows thus a flexible illumination or projection of information on the object in the field of view of a surgical microscope, which leads to information enrichment during surgical procedures.

By replacing simple light sources previously used in connection with a microscope or adding an additional "intelligent" light source or beamer being for example a LCD beamer, a xenon lamp or a laser projector, different light setups and displays of additional information directly on the surface of the object being observed through the microscope can be provided.

The surgical microscope comprises according to a first embodiment a light source, such as a laser projector or LCD-beamer, which generates light within the microscope or which light can be transmitted to the microscope using for example an optical fiber. This light is projected onto the surface of the object to be viewed by the microscope using preferable the objective lense of the microscope to project, emit or focus the light generated by this light source onto the object. Furthermore, a control unit is provided to generate signals or images to be projected onto the object to control the light source of the surgical microscope, so that the images projected onto the object are preferably in a predetermined or corresponding spatial relationship to the object preferably matching the structures or surface of the viewed object. Thus, a structured illumination can be provided.

The light emitted by the light source can be guided to the object of the microscope using preferably a prism and/or a semi-transparent mirror being present in the microscope, which prism or semi-transparent mirror can also be used for deflecting or guiding the image of the viewed object to the ocular of the microscope to be seen by the observer. The surgical microscope consists according to a second embodiment of an optics carrier, a stand, a light source that can be built by an external laser projector, and a control unit.

One or more cameras that observes the field of view of the microscope and a surgical navigation system are added for either embodiment to control the projector itself or to control the control unit.

For the advanced features in combination with the navigation system as set forth below, the microscope and preferably the optional camera(s) and the projector have to be calibrated to allow the spatially correct display of information.

The following setups could be imagined and of course others could be possible:

Plain light: similar to the conventional microscope light, monochrome light, adjustable in color and intensity, can be emitted. The color adjustment could for example be used to accentuate specific tissue.

Simple shaped light: Like an advanced aperture, the light cannot only be restricted having a circular shape, but for every arbitrary shape at the outline the projector can handle. This can be useful to blend out useless information, e.g. in neurosurgery the light can be adjusted to just enlighten the craniotomy.

Complex shaped light: Different parts of the field of view can be enlightened with different light parameters, e.g. different colors and/or different brightness.

Information enrichment: The light source, e.g. xenon lamp, bamer (having e.g. a built-in LCD-device) or laser projector, can be used to project information (e.g. text, images, contours, ...) directly onto the object (e.g. a human brain) that is watched through the microscope. This is similar to the built in displays also known as Head-up Displays (HUD) of modem surgical microscopes but has some advantages.

A conventional microscope HUD is inconvenient to handle for some surgeons because of the different distances of the HUD and the real life object to the surgeon's eye. Some surgeons can compensate for that but some cannot. With the integrated laser projector this problem does not occur because the information is displayed directly on the real life object.

A conventional microscope HUD has very limited contrast because the built in light source of the microscope outshines the information displayed on the HUD. Because of the combination of the light source and the information display, this invention allows to display the information with a high contrast without darkening the whole field of view by dimming the light source.

By combining this setup with an optionally color and/or spatially calibrated video camera that records the field of view of the microscope, the video information is used to automatically adjust the light color depending on the tissue color. When two cameras are used (or one camera at different positions) the three dimensional structure of the field of view can be determined. This information can be incorporated in the algorithms controlling the projector (e.g. projecting information related to the spatial position of the part of the object part of the information is projected on).

The combination with the video camera can also be used to display images that have been recorded beforehand, e.g. the image of the object in the field of view of the microscope is recorded at special lighting conditions (e.g. tumor markers in fluorescent light) and this information is redisplayed later at different lighting conditions (e.g. "normal" light) to combine multiple lighting conditions in one.

When the information display of the projector is separated for the left and right ocular (and therefore for the left and right eye), also a stereoscopic projection is possible. One possible implementation would be two shutters integrated in the oculars where at each point in time only one shutter is open and at the same point in time the projector generates the corresponding image for the open ocular.

This device can be combined with a surgical navigation system to display anatomical data on the real world object at the correct position. The information available by the navigation system can be used to adapt the projector. For example the information of the shape of the head of the patient and the information of the position of this head in relation to the (calibrated) microscope and therefore to the (calibrated) projector can be used to adapt the projected shape.

The laser projector could also be used as an additional component to be added to a conventional microscope in addition to the conventional light source. This would not provide the full power as the replacement of the standard light source regarding light shaping etc., but the information overlay over the real world object is still advantageous compared to a conventional HUD e.g. regarding the accommodation problem.

The laser can be tuned to frequencies that meet the resonance of diagnostically or treatment related applied flourescent markers, so that these markers selectively light up and/or are activated.

The laser can be tuned to an energy where it can be used for heating up the tissue (coagulation, ablation....).

The invention is hereafter described with reference to the accompanying drawings in which:
FIG. 1 is a block diagram of a system embodying the present inventive concepts and including a reference system and an operating microscope;
FIG. 2 shows a schematic design of a monocular microscope with an add-on shader lamp or beamer, and
FIG. 3 shows a schematic design of a monocular microscope with a laser beamer according to a further embodiment.

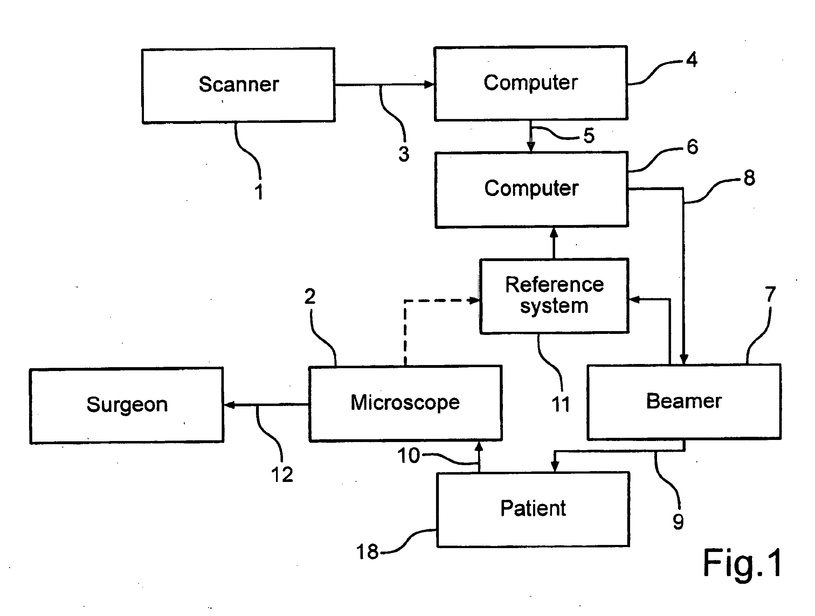

FIG. 1 shows a block diagram of an embodiment of the present invention. The reference display system receives information from a three-dimensional imaging device 1 (e.g., a CT, an MRI, or a PET scanner or an ultrasonic imaging device) and includes an operating microscope 2. The function of the system is to extract information concerning a region of the brain of the patient 18 by a CT scan, for example, and to present that information in the form of an image projected onto the patient 18 and especially onto an object in the field of view of the microscope 2 during an operation, at the same time as the same region of the object or brain 18 is displayed as an optical image which can be seen as a combined image 12 by the neurosurgeon looking through the microscope 2. In this way the surgeon can view simultaneously the actual brain region 18 as well as a reconstruction of the brain region which is projected onto and displayed on the head or brain surface 18 as an optical picture or signal 9 and which highlights e.g. a problem area, such as a tumor, which is to be removed.

To simplify this explanation, the three-dimensional imaging device 1 will be considered to be images from a CT scanner. The interacting elements in the system serve to project and optionally display three-dimensional anatomical and/or pathological information derived from the brain, or other organ or body part, by the scanner 1 on a part of the surface of the patient as visualized through the microscope 2 to present a composite view 12 to the surgeon viewing the patient 18 with the image 9 projected thereon.

The scanner 1 provides image information that is stored, e.g., on x-ray film or digital tape or by a first computer 4. If not in digital form, the information is digitized and the digitized information is connected as input at 3 to the first computer 4 that acts as a treatment planning computer that provides an output 5 that is transferred to a second computer 6. The computers 4 and 6 can be the same or different computers. The scanner 1 can provide real time data to the computer 4, but, typically, the information is stored on magnetic tape and, later, it is introduced to the computer 4 for processing. The computer 6 takes the information from the computer 4 and information from reference system 11, such as e.g. VectorVision® provided by BrainLAB®, that identifies the spatial position of the patient 18 having e.g. markers 18a. The computer 6 is programmed to reformat the digitized information taking into account the relative position between an optical display system or beamer 7 and the patient 18 and to provide an output signal at 8 representing the reformatted information. The information at 8 eventually is fed to the optical display system 7 (e.g. laser beamer (monochrome or colour) or any other optical projecting means) which forms an image that is displayed on the surface of the patient 18.

Assuring that the image of the brain region generated by the computer 6 and displayed by laser beamer 7 registers with the surface of the patient 18 is an important aspect of the present invention, as discussed later.

Whereas the signals at 3, 5, and 8 are electrical signals (i.e., either analog or binary), the signal output at 9 of the laser beamer 7 is an optical signal which conveys the reformatted information as an image directly to the patient 18. The neurosurgeon can see both images 9 and 18 as combined image 12 by only viewing the patient 18 since they are superposed upon one another.

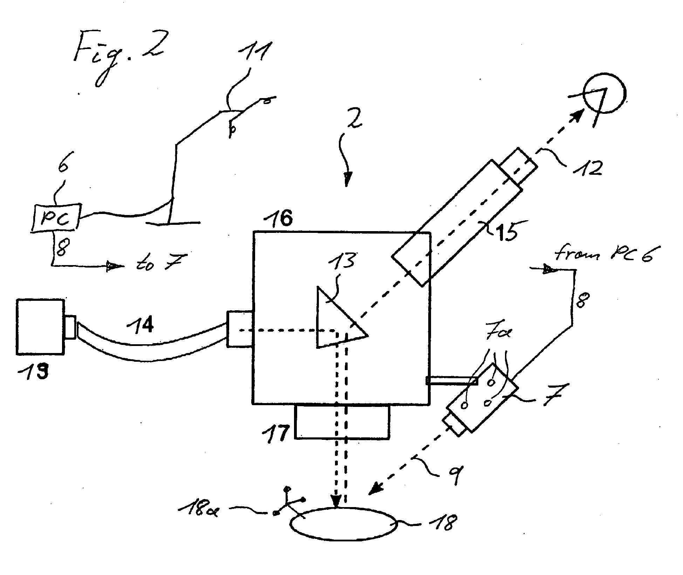

The computer-generated image projected by beamer 7 is displayed on the patient 18 and provides one combined image 12 that the surgeon sees. The image should be bright enough to provide an acceptable superposed image on the patient 18 or object in the illuminated operative field. The laser beamer 7 also has size constraints, since it is preferably mounted on the microscope 2, as seen in Fig. 2.

The beamer 7 can e.g. be attached to a standard operating room microscope 2 so that the beamer 7 is able to generate an optical image 9 on an object 18 in the field of view of the microscope 2.

The micrsoscope 2 has an ocular 15, as shown in Fig. 2, through which a surgeon can see the combined images 12 being a combination of an image of the object 18 viewed through objective lense 17 and image 9 projected onto object 18 by the laser beamer 7 connected to the microscope 2.

Reference numeral 16 designates a magnification changer containing a prism or semi-transparent mirror 13 receiving light to illuminate the object 18 from a light source 19, as for example a xenon lamp, transmitted through an optical fibre 14 and deflected by the prism 13 onto the object 18. Prism or semi-transparent mirror 13 also serves to deflect the combined image 12 to be sent to ocular 15 viewed by the surgeon.

It will be appreciated, on the basis of the foregoing explanation, that the computer-generated image of the brain region of the patient can be precisely focussed and projected onto the surface of head or brain 18, because the beamer 7 is rigidly attached to the microscope 2. It remains, therefore, to bring-the optical image of that same brain region to the projection plane of beamer 7 being the surface of the object 18 to effect registration of the images with object 18. This requires precise knowledge as to the spatial location of the beamer 7 or the microscope 2 carrying beamer 7 and of the patient or object 18 relative to the beamer 7 or microscope 2, as explained below.

A scheme for registering the projection of a computer-generated image of an object with the surface of the same part of the object 18, e.g. brain (or other body part) and viewed in the microscope 2 is described below. The aim is to present to the surgeon a computer-generated or reformatted image (from a CT scan or other imaging system) projected in proper orientation and scale upon the object 18, e.g. the brain. Thus, establishing the spatial relationship among the object or patient 18, and the focal or projection plane of the beamer 7, is important.

Exactly projecting the images is accomplished by spatially relating both the imaging data and the beamer 7 (or the operating microscope 2 with attached beamer 7) to the same points (hereinafter called markers) 18a on the patient 18. The general registration procedure can involve CT scanning the patient 18 with at least three markers 18a attached to the head of the patient 18 (the patient may be supine or seated). The markers 18a are composed of a material (e.g., tungsten), that is physically detectable by the imaging system (i.e., the CT scan at this part of the explanation) during scanning as well as visually to the microscope 2 or an IR-camera 11 to achieve registration of the computer-generated projected image from the CT scan with the patient 18 being preferably at the focal plane of the microscope 2.

A preferred way to establish the spatial relationship between the beamer 7 (or microscope 2) and the markers 18a, as well as to track any subsequent movement of the microscope 2 or beamer 7, includes optical tracking of the markers 18a attached to the object or patient 18 and markers 7a attached to the beamer 7 and/or microscope 2. Determining the spatial relationship of two objects 7 and 18 each bearing markers 7a and 18a is well known in the art.

The reference system 11, such as the mentioned VectorVision® system, can determine the position of the microscope 2 or beamer 7 and its focal plane with respect to the patient's head 18 and CT scans.

The reconstructed CT scan, as above indicated, must be projected by beamer 7 as a two-dimensional CRT image. This involves converting the reconstructed slice from a matrix of image data in three coordinates to one of two coordinates (x, y). A microscope or beamer coordinate system could represent the projection or focal plane as x and y, normal to the optical axis with the origin at the focal point. This technique requires a transformation of coordinates because the beamer or microscope coordinate system will be constantly changing with respect to the location of the object 18 and corresponding CT scan data of the object 18 as the surgeon moves the microscope 2. Regardless of the reference frame used for reconstructing the slice, in order to project and thus display the proper image, the slice must be transformed into beamer or microscope coordinates. The advantage of transforming all the data and performing the calculations in the microscope (with attached beamer) coordinate system is that if the surgeon moves the microscope 2 only slightly or along the optical axis, the reconstruction calculations can be greatly reduced and allow the surgeon to quickly call up new slices for projection.

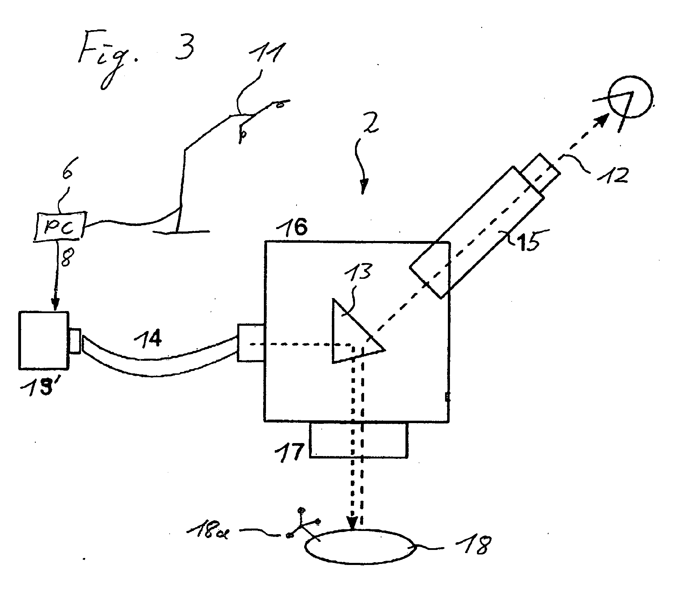

Fig. 3 shows a further embodiment similar to the embodiment described with reference to Fig. 2, wherein the same or similar elements are designated with the same reference numerals.

Unlike the embodiment shown in Fig. 2, the external laser beamer 7 is omitted and the single light or illumination source is constituted by laser beamer 19' which is connected to the microscope 2 via fiber 14 but which could also be integrated directly into the microscope 2. The laser beamer 19' receives the information 8 from the computer 6 and outputs laser light, which enters the prism or semi-transparent mirror 13 to be projected directly onto the surface of the object 18 through the objective lens 17.

Thus, an external or separate light source, such as laser beamer 7 shown in the embodiment of Fig. 2, can be omitted. The image to be displayed on the surface of the object 18 can be projected from the microscope 2 itself using preferably the optics of the microscope 2.

## Claims

1. A method of integrating image information (3, 5, 8, 9) from an external source, e.g. an imaging device (1), and an operating microscope (2) before or during an operative procedure on an object (18) or body parts of a patient comprising: positioning an operating microscope (2) before or in the course of said operative procedure at an operative location relative to the patient (18); establishing the spatial relationship among the image information, the patient (18), and the focal plane of a projector (7) or the projection space or the projector (7) itself; introducing the image information and spatial relationship to a computer and reformatting the image information to generate a computer-generated image of the body part at a determined area or plane related to or directly on the surface of the object (18); and projecting the computer-generated image onto the object (18) or patient for coordinated viewing (12) of the computer-generated image and the patient, **characterised in that** the field of view of the operating microscope (2) is recorded with an optionally color and/or spatially calibrated video camera and **in that** the video information is used to adjust the colors for the projected image depending on the tissue color to accentuate specific tissue.

2. A method according to claim 1 that includes providing a plurality of markers (7a, 18a) which are physically detectable by a reference system (11) to a projector or beamer (7) and to an object (18) to permit detection of the relative position between the projector or beamer (7) and the object (18).

3. A method according to claim 1 or 2 comprising establishing the spatial relationship between the object (18), the projector (7) and the imaging information (3) using a reference system (11), detecting markers (7a, 18a) and calculating a preferably two-dimensional projection image from the imaging information (3).

4. A method according to one of the claims 1 to 3, wherein the three dimensional structure of the surface of the object (18) is determined using one camera viewing the object from different positions or two or more cameras to adapt or modify the picture to be projected on the determined surface structure.

5. A reference display system to receive information from an imaging system (1) that extracts three-dimensional information about a part of the body (18) of a patient, the system comprising: an operating microscope (2) being preferably calibrated and being positioned in an operative location relative to the patient (18); means (11) for establishing the spatial relationship among the imaging system information, the patient (18), and the projection plane of a projector (7) or the projection space and/or the projector (7) itself, the projector being preferably calibrated; computer means connected to receive the information from the imaging system (1) and from said means (11) for establishing the spatial relationship and programmed to reformat the information from the imaging system to provide an output signal representative of a computer-generated image corresponding to a determined plane having a predetermined relationship with the projection plane of the projector (7) or being on the surface of the body (18); **characterised by** an optionally color and/or spatially calibrated video camera that records the field of view of the operating microscope (2); and means (7) to project the computer-generated image onto the part of the body (18) for coordinated viewing of the computer-generated projected image (9) and the patient or part of the body (18) through the operating microscope (2), the means (7) being configured to use the video information to adjust the colors of the projected image depending on the tissue color to accentuate specific tissue.

6. Apparatus that includes the reference display system according to claim 5 and that further includes an imaging system in the form of a CT or resonance imaging (MRI) scanner or positron emission tomography (PET) scanner or an ultrasound imaging device.

7. A system according to claim 5 or 6 in which the means (11) for establishing the spatial relationship comprises two IR-cameras whose output is digitized.

8. A system according to claim 5, 6 or 7 that includes a plurality of markers (7a, 18a) which are physically detectable by the cameras and/or the imaging system to permit determining the relative position between the projector (7) and the object (18).

9. A system according to claim 8, wherein the markers (18a) are spatially fixed with respect to the patient (18) to permit accurate location of anatomic and/or pathologic structures of interest.

10. A system according to one of claims 5 to 9 in which the appropriate reformatted image at the determined plane, based on the imaging system information, is displayed or projected by a laser beamer (7) which preferably is mounted on the operating microscope (2) in a way that a person looking through the operating microscope (2) sees both the operative field and the image projected onto the object (18).

11. A system according to claim 10 wherein the appropriate reformatted image is determined by the position of the microscope image plane or microscope (2).

12. A system according to one of the claims 5 to 11 comprising at least one fixed or moveable camera to determine the three-dimensional structure of the surface of the object (18).

## Patentansprüche

1. Verfahren zum Integrieren einer Bildinformation (3, 5, 8, 9) von einer externen Quelle, zum Beispiel einem bildgebenden Gerät (1), und einem Operationsmikroskop (2) vor oder während eines operativen Verfahrens an einem Objekt (18) oder Körperteilen eines Patienten umfassend: Positionieren eines Operationsmikroskops (2) vor oder im Verlauf des operativen Verfahrens bei einer Operationsstelle relativ zu dem Patienten (18); Festlegen des räumlichen Verhältnisses zwischen der Bildinformation, dem Patienten (18) und der Focus-Ebene eines Projektors (7) oder dem Projektionsraum des Projektors (7) selbst; Eingeben der Bildinformation und des räumlichen Verhältnisses zu einem Computer und umformatieren der Bildinformation, um ein computer-erzeugtes Bild des Körperteils bei einem bestimmten Bereich oder einer Ebene im Verhältnis zu oder direkt auf der Oberfläche des Objektes (18) zu erzeugen; und projizieren des computer-erzeugten Bildes auf das Objekt (18) oder den Patienten zum koordinierten Betrachten (12) des computer-generierten Bildes und des Patienten, **dadurch gekennzeichnet, dass** das Blickfeld des Operationsmikroskops (2) mit einer optional farb- und/oder räumlich kalibrierten Videokamera aufgezeichnet wird und dass die Videoinformation verwendet wird, um die Farben für das projizierte Bild in Abhängigkeit von der Gewebefarbe einzustellen, um bestimmtes Gewebe hervorzuheben.

2. Verfahren nach Anspruch 1, welches das Vorsehen einer Mehrzahl von Markern (7a, 18a) umfasst, welche physikalisch durch ein Referenzsystem (11) detektierbar sind, an einem Projektor oder Beamer (7) und an einem Objekt (18), um die Detektion der relativen Position zwischen dem Projektor oder Beamer (7) und dem Objekt (18) zu ermöglichen.

3. Verfahren nach Anspruch 1 oder 2 umfassend das Festlegen des räumlichen Verhältnisses zwischen dem Objekt (18), dem Projektor (7) und der Abbildungsinformation (3) unter Verwendung eines Referenzsystems (11), Detektieren der Marker (7a, 18a) und Berechnen eines vorzugsweise zwei-dimensionalen Projektionsbildes aus der Abbildungsinformation (3).

4. Verfahren nach einem der Ansprüche 1 bis 3, wobei die dreidimensionale Struktur der Oberfläche des Objekts (18) bestimmt wird unter Verwendung einer Kamera, welche das Objekt aus verschiedenen Positionen betrachtet oder zwei oder mehr Kameras, um das auf die bestimmte Oberflächenstruktur zu projizierende Bild zu adaptieren oder zu modifizieren.

5. Referenz-Anzeige-System, um eine Information von einem Bildgebungssystem (1) zu empfangen, welches eine drei-dimensionale Information über einen Teil des Körpers (18) eines Patienten extrahiert, wobei das System aufweist: ein Operationsmikroskop (2), welches vorzugsweise kalibriert ist und an einer Operationsstelle relativ zu dem Patienten (18) angeordnet ist; eine Vorrichtung (11) zum Festlegen des räumlichen Verhältnisses zwischen der Bildgebungssysteminformation, dem Patienten (18), und der Projektionsebene eines Projektors (7) oder der Projektionsebene und/oder dem Projektor (7) selbst, wobei der Projektor vorzugsweise kalibriert ist; eine Computervorrichtung, welche verbunden ist, um die Information von dem Bildgebungssystem (1) und von der Vorrichtung (11) zu empfangen, zum Festlegen des räumlichen Verhältnisses und programmiert ist, um die Information von dem Bildgebungssystem umzuformatieren, um ein Ausgangssignal zur Verfügung zu stellen, welches ein computer-generiertes Bild darstellt, korrespondierend zu einer bestimmten Ebene mit einem vorgegebenen Verhältnis zu der Projektionsebene des Projektors (7) oder welches auf der Oberfläche des Körpers (18) liegt; **gekennzeichnet durch** eine optional farb- und/oder räumlich kalibrierte Videokamera, welche das Blickfeld des Operationsmikroskops (2) aufzeichnet; und eine Vorrichtung (7), um das computer-generierte Bild auf den Teil des Körpers (18) zu projizieren, zum koordinierten Betrachten des computer-generierten projizierten Bildes (9) und des Patienten oder Teils des Körpers (18) **durch** das Operationsmikroskop (2), wobei die Vorrichtung (7) konfiguriert ist, um die Videoinformation zu verwenden, um die Farben des projizierten Bildes in Abhängigkeit von der Gewebefarbe einzustellen, um bestimmtes Gewebe hervorzuheben.

6. Vorrichtung, welche das Referenz-Anzeige-System nach Anspruch 5 umfasst und weiter ein Bildgebungssystem aufweist in der Gestalt eines CT oder Resonanzbildgebungs- (MRI) Scanners oder eines Positron-Emissions-Tomographie- (PET) Scanners oder einer Ultraschallbildgebungsvorrichtung.

7. System nach Anspruch 5 oder 6, wobei die Vorrichtung (11) zum Festlegen des räumlichen Verhältnisses zwei IR-Kameras aufweist, deren Ausgang digitalisiert ist.

8. System nach Anspruch 5, 6 oder 7, welches eine Mehrzahl von Markern (7a, 18a) aufweist, welche physikalisch detektierbar sind durch die Kameras und/oder das Bildgebungssystem, um das Bestimmen der relativen Position zwischen dem Projektor (7) und dem Objekt (18) zu ermöglichen.

9. System nach Anspruch 8, wobei die Marker (18a) räumlich fest sind in Bezug auf den Patienten (18), um das genaue Lokalisieren von anatomischen und/oder pathologischen Strukturen von Interesse zu ermöglichen.

10. System nach einem der Ansprüche 5 bis 9, wobei das geeignet umformatierte Bild auf der bestimmten Ebene, basierend auf der Bildgebungssysteminformation, angezeigt oder projiziert wird durch einen Laser-Beamer (7), der vorzugsweise auf dem Operationsmikroskop (2) so montiert ist, dass eine Person, welche durch das Operationsmikroskop (2) schaut, beides sieht, das Operationsfeld und das auf das Objekt (18) projizierte Bild.

11. System nach Anspruch 10, wobei das geeignet umformatierte Bild durch die Position der Mikroskop-Bildebene oder des Mikroskops (2) bestimmt ist.

12. System nach einem der Ansprüche 5 bis 11 mit mindestens einer feststehenden oder bewegbaren Kamera, um die drei-dimensionale Struktur der Oberfläche des Objekts (18) zu bestimmen.

## Revendications

1. Procédé d'intégration d'une information d'image (3, 5, 8, 9) à partir d'une source extérieure, par exemple un dispositif d'imagerie (1), et d'un microscope fonctionnel (2) avant ou durant une procédure opératoire sur un objet (18) ou des parties corporelles d'un patient, comprenant : le positionnement d'un microscope fonctionnel (2) avant ou au cours de ladite procédure opératoire en un emplacement opératoire par rapport au patient (18) ; l'établissement de la relation spatiale entre l'information d'image, le patient (18) et le plan focal d'un projecteur (7) ou l'espace de projection ou le projecteur (7) lui-même ; l'introduction de l'information d'image et de la relation spatiale dans un ordinateur et le reformatage de l'information d'image de façon à générer une image générée par ordinateur de la partie corporelle dans une zone ou un plan prédéterminé associé à la surface de l'objet (18) ou directement sur celle-ci ; et la projection de l'image générée par ordinateur sur l'objet (18) ou le patient pour une visualisation coordonnée (12) de l'image générée par ordinateur et du patient, **caractérisé en ce que** le champ de vision du microscope fonctionnel (2) est enregistré avec une caméra vidéo étalonnée dans l'espace et/ou, de façon optionnelle, en couleurs, et **en ce que** l'information vidéo est utilisée pour ajuster les couleurs pour l'image projetée en fonction de la couleur des tissus afin d'accentuer un tissu spécifique.

2. Procédé selon la revendication 1, qui comprend la disposition d'une pluralité de marqueurs (7a, 18a) qui peuvent être physiquement détectés par un système de référence (11) sur un projecteur ou un dispositif de réalisation de faisceau (7) et sur un objet (18) de façon à permettre la détection de la position relative entre le projecteur ou le dispositif de réalisation de faisceau (7) et l'objet (18).

3. Procédé selon la revendication 1 ou 2, comprenant l'établissement de la relation spatiale entre l'objet (18), le projecteur (7) et l'information d'imagerie (3) à l'aide d'un système de référence (11), la détection de marqueurs (7a, 18a) et le calcul d'une image de projection, de préférence en deux dimensions, à partir de l'information d'imagerie (3).

4. Procédé selon l'une des revendications 1 à 3, dans lequel la structure en trois dimensions de la surface de l'objet (18) est déterminée à l'aide d'une caméra visualisant l'objet à partir de positions différentes ou de deux ou de plusieurs caméras, de façon à adapter ou à modifier l'image devant être projetée sur la structure de surface déterminée.

5. Système d'affichage de référence pour recevoir une information à partir d'un système d'imagerie (1) qui extrait une information en trois dimensions concernant une partie du corps (18) d'un patient, le système comprenant : un microscope fonctionnel (2), qui est de préférence étalonné et qui est positionné dans un emplacement opératoire par rapport au patient (18) ; des moyens (11) pour établir la relation spatiale entre l'information de système d'imagerie, le patient (18) et le plan de projection d'un projecteur (7) ou l'espace de projection et/ou le projecteur (7) lui-même, le projecteur étant de préférence étalonné ; des moyens informatiques connectés de façon à recevoir l'information à partir du système d'imagerie (1) et à partir desdits moyens (11) de façon à établir la relation spatiale et programmés afin de reformater l'information à partir du système d'imagerie de façon à délivrer un signal de sortie représentatif d'une image générée par ordinateur correspondant à un plan prédéterminé ayant une relation prédéterminée avec le plan de projection du projecteur (7) ou se trouvant sur la surface du corps (18) ; **caractérisé par** une caméra vidéo étalonnée dans l'espace et/ou, de façon optionnelle, en couleurs, qui enregistre le champ de vision du microscope fonctionnel (2) ; et des moyens (7) pour projeter l'image générée par ordinateur sur la partie du corps (18) pour une visualisation coordonnée de l'image projetée générée par ordinateur (9) et le patient ou la partie du corps (18) par l'intermédiaire du microscope fonctionnel (2), les moyens (7) étant configurés de façon à utiliser l'information vidéo pour ajuster les couleurs de l'image projetée en fonction de la couleur des tissus afin d'accentuer un tissu spécifique.

6. Appareil qui comprend le système d'affichage de référence selon la revendication 5, et qui comprend de plus un système d'imagerie sous la forme d'un scanneur de tomographie informatisée ou d'imagerie à résonance (IRM), ou un scanneur de tomographie à émission de positrons (PET) ou un dispositif d'imagerie à ultrasons.

7. Système selon la revendication 5 ou 6, dans lequel les moyens (11) pour établir la relation spatiale comprennent deux caméras à infrarouges dont la sortie est numérisée.

8. Système selon la revendication 5, 6 ou 7, qui comprend une pluralité de marqueurs (7a, 18a) qui peuvent être physiquement détectés par les caméras et/ou le système d'imagerie, de façon à permettre la détermination de la position relative entre le projecteur (7) et l'objet (18).

9. Système selon la revendication 8, dans lequel les marqueurs (18a) sont fixés dans l'espace par rapport au patient (18), de façon à permettre une localisation précise de structures anatomiques et/ou pathologiques auxquelles on s'intéresse.

10. Système selon l'une des revendications 5 à 9, dans lequel l'image reformatée appropriée dans le plan déterminée, en fonction de l'information de système d'imagerie, est affichée ou projetée par un dispositif de réalisation de faisceau de laser (7), qui est de préférence monté sur le microscope fonctionnel (2) de telle sorte qu'une personne regardant à travers le microscope fonctionnel (2) voie tout à la fois le champ opératoire et l'image projetée sur l'objet (18).

11. Système selon la revendication 10, dans lequel l'image reformatée appropriée est déterminée par la position du plan d'image du microscope ou par le microscope (2).

12. Système selon l'une des revendications 5 à 11, comprenant au moins une caméra fixe ou mobile pour déterminer la structure en trois dimensions de la surface de l'objet (18).
